# EUROPEAN PATENT APPLICATION

(11) **EP 4 353 211 A1**
(43) Date of publication of application: **17.04.2024**
(21) Application number: 21951957.6
(22) Date of filing: 13.12.2021
(51) Int. Cl.: A61F 13/49, A61F 13/532

(54) **PANTS-TYPE ABSORBENT ARTICLE**

(30) Priority: 30.07.2021 CN 202110870106
(71) Applicant: Unicharm Corporation, Ehime 799-0111 (JP)
(72) Inventor: UDAKA, Hikari, Kanonji-shi, Kagawa 769-1602 (JP); FUJIMOTO, Kazuya, Kanonji-shi, Kagawa 769-1602 (JP); ITO, Yoshihiro, Kanonji-shi, Kagawa 769-1602 (JP)
(74) Representative: Staeger & Sperling Partnerschaftsgesellschaft mbB
(86) International application number: PCT/JP2021/045861
(87) International publication number: WO 2023/007764

(57) **Abstract**

The present invention provides a pants-type absorbent article (1) having a belt part (32) and an absorbent body (10) containing pulp and/or a high-absorbency polymer, the pants-type absorbent article (1) being provided with a waist opening (BH) and a pair of leg openings (LH), the belt part (32) being joined to the absorbent body (10) by joining parts (40) inclined, from the waist opening (BH) towards the leg openings (LH), downwards from above with respect to the vertical direction and outwards from the inside with respect to the lateral direction, wherein the absorbent body (10) has, with respect to the lateral direction in a state in which the absorbent body (10) is stretched, a different-basis-weight section (50) in which the total basis weight of the high-absorbency polymer and the pulp contained per unit area is different from that in adjacent portions.

## Description

### FIELD

The present invention relates to an underpants-shaped absorbent article.

### BACKGROUND

An underpants-shaped absorbent article has been conventionally widely known in which the absorbent main body is joined to the central portion of the front and back waist belt portions in the lateral direction. For example, Patent Document 1 discloses an underpants-shaped diaper 1 which includes a front band member 2A bonded to the front side of the absorbent main body 3 and a back band member 2B bonded to the back side of the absorbent main body 3, and which is formed into an underpants shape by bonding the front band member 2A and the back band member 2B in the side portions 1a.

### [CITATION LIST]

### [PATENT LITERATURE]

[Patent Document 1] Japanese Patent Application Publication No. 2004-329238

### SUMMARY

### [TECHNICAL PROBLEM]

A conventional underpants-shaped absorbent article (diaper) as in Patent Document 1 has a configuration as follow: when the waist belt portion is pulled to the one side in the lateral direction during putting on and the like, the absorbent main body, which is connected to the waist belt portion, is likely to be pulled together towards the one side in the lateral direction. In this case, that the absorbent main body is deformed as a whole or misaligned causes a problem of impairing the fit.

The present invention was achieved in light of conventional problems such as that described above, an aspect of the present invention is to provide an underpants-shaped absorbent article in which the fit of an absorbent main body is good during usage.

### [SOLUTION TO PROBLEM]

A main aspect of the present invention of achieving the above-described aspect is an underpants-shaped absorbent article having a vertical direction, a lateral direction, and a front-back direction that intersect with each other, the underpants-shaped absorbent article including: an absorbent main body that includes at least one of pulp and superabsorbent polymer; and a belt portion that connects a front upper end portion and a back upper end portion of the absorbent main body, the underpants-shaped absorbent article having a waist opening and a pair of leg openings, the belt portion being joined to the absorbent main body by a joining portion that inclines from the waist opening towards the leg opening from above to below in the vertical direction and from inside to outside in the lateral direction, in a state where the absorbent main body is stretched, the absorbent main body having a basis-weight different portion, the basis-weight different portion being a portion in which a total basis weight of pulp and superabsorbent polymer that are contained per the unit area is different compared to a portion adjacent in the lateral direction.
Features of the present invention other than the above will become clear by reading the description of the present specification with reference to the accompanying drawings.

### [ADVANTAGEOUS EFFECT OF THE INVENTION]

According to the present invention, it is possible to provide an underpants-shaped absorbent article in which the fit of an absorbent main body is good during usage.

### BRIEF DESCRIPTION OF DRAWINGS

FIG. 1 is a schematic front view of a diaper 1 as seen from a front side.
FIG. 2A is a plan view of the diaper 1 in a state where an absorbent main body 10 and a belt portion 32 are stretched in a longitudinal direction.
FIG. 2B is a cross-sectional view of the diaper 1 in the state shown in FIG. 2A, taken along a line A-A.
FIG. 3A is a plan view of the diaper 1, which is unfolded by separating first joining portions 41 and second joining portions 42 in FIG. 2A.
FIG. 3B is a cross-sectional view of the diaper 1 in the state shown in FIG. 3A, taken along a line B-B.
FIG. 4A is a schematic plan view of an absorbent main body 10 in the stretched state as seen from a skin side.
FIG. 4B is a cross-sectional view of the absorbent main body 10 taken along a line C-C in FIG. 4A.
FIGS. 5A and 5B are diagrams illustrating another example of a basis-weight different portion 50.
FIG. 6 is a diagram illustrating the difference in fit of the absorbent main body 10 during usage, between a conventional underpants-shaped diaper and the underpants-shaped diaper 1 of the present embodiment.
FIGS. 7Ato 7C are diagrams illustrating modified examples of the basis-weight different portion 50.
FIGS. 8A and 8B are diagrams illustrating examples of a case in which compressed portions 60 are provided in the absorbent main body 10.
FIG. 9 is a schematic plan view illustrating the arrangement of the basis-weight different portion 50 on the back side of the diaper 1.

### DESCRIPTION OF EMBODIMENTS

At least the following matters will be clear with the description of this specification and the attached drawings.

An underpants-shaped absorbent article having a vertical direction, a lateral direction, and a front-back direction that intersect with each other, the underpants-shaped absorbent article including: an absorbent main body that includes at least one of pulp and superabsorbent polymer; and a belt portion that connects a front upper end portion and a back upper end portion of the absorbent main body, the underpants-shaped absorbent article having a waist opening and a pair of leg openings, the belt portion being joined to the absorbent main body by a joining portion that inclines from the waist opening towards the leg opening from above to below in the vertical direction and from inside to outside in the lateral direction, in a state where the absorbent main body is stretched, the absorbent main body having a basis-weight different portion, the basis-weight different portion being a portion in which a total basis weight of pulp and superabsorbent polymer that are contained per the unit area is different compared to a portion adjacent in the lateral direction.

According to the underpants-shaped absorbent article, the force acting on the absorbent main body from the one side to the other side in the lateral direction is distributed or weakened by the basis-weight different portion, and this makes the force less likely to be transmitted to the opposite side in the lateral direction. Therefore, even if the lateral one-side portion of the absorbent main body is pulled to the other side, the lateral other-side portion, which is located on the opposite side across the basis-weight different portion, is less likely to be pulled to the one side, and this can make it less likely to cause displacement and deformation. As a result, it is possible to realize the underpants-shaped absorbent article that has a better fit of the absorbent main body during usage.

In such an underpants-shaped absorbent article, it is desirable that the underpants-shaped absorbent article has a conjunction-suppression region that is composed of the one or more basis-weight different portion, and that a length of the conjunction-suppression region in the vertical direction is longer than a length of the conjunction-suppression region in the lateral direction.

According to the underpants-shaped absorbent article, in the lateral direction of the absorbent main body, the one-side portion and the other-side portion that are separated by the conjunction-suppression region are likely to be widely formed in the longitudinal direction. As a result, the deformation and displacement of the absorbent main body are less likely to be transmitted on the counter side in the lateral direction, making it possible to suppress deterioration of the fit during usage.

In such an underpants-shaped absorbent article, it is desirable that the basis-weight different portion has a portion that is continuous in the vertical direction.

According to the underpants-shaped absorbent article, the basis-weight different portion is continuous in the longitudinal direction, and therefore the left and right conjunction of the absorbent main body is more likely to be suppressed compared to the case where the basis-weight different portion is not continuous. This suppresses the deterioration of the fit of the absorbent main body when the diaper 1 is put on.

In such an underpants-shaped absorbent article, it is desirable that the absorbent main body has a plurality of the basis-weight different portions that are arranged spacing in the vertical direction.

According to the underpants-shaped absorbent article, a gap is formed between the basis-weight different portions which are adjacent to each other in the vertical direction, and this makes the absorbent main body likely to deform in the longitudinal direction (vertical direction) from the starting point of the bend, which is the stiffness difference that occurs in the gap. This makes the absorbent main body likely to flexibly deform according to projections/recessions of the wearer's body, and the fit can be improved.

In such an underpants-shaped absorbent article, it is desirable that the basis-weight different portion is provided in a central portion in the lateral direction, and that, in the basis-weight different portion, a total basis weight of pulp and superabsorbent polymer that are contained per unit area is higher than a portion that is adjacent to the central portion on both sides in the lateral direction.

According to the underpants-shaped absorbent article, in the lateral central portion, the portion where the basis weight of the absorbent main body is higher (the basis-weight different portion) serves as a bending point, making the absorbent main body be folded one time in the lateral direction. This makes the absorbent main body likely to fit into the groove of the wearer's buttocks and the excretion opening, and the like. This makes it possible to improve the fit of the absorbent main body and simultaneously to makes excretion leakage less likely to occur.

In such an underpants-shaped absorbent article, it is desirable that the basis-weight different portion is provided in a central portion in the lateral direction, and that, in the basis-weight different portion, a total basis weight of pulp and superabsorbent polymer that are contained per unit area is lower than a portion that is adjacent to the central portion on both sides in the lateral direction.

According to the underpants-shaped absorbent article, in the lateral central portion, the portion where the basis weight of the absorbent main body is lower (the basis-weight different portion) serves as a bending point, making the absorbent main body be folded one time in the lateral direction. This makes the absorbent main body likely to fit into the groove of the wearer's buttocks and the excretion opening, and the like. This makes it possible to improve the fit of the absorbent main body and simultaneously to makes excretion leakage less likely to occur.

In such an underpants-shaped absorbent article, it is desirable that the absorbent main body includes a liquid-absorbent absorbent core, and that a compressed portion is provided on each of two lateral sides of the basis-weight different portion, the compressed portion being a portion that has a predetermined length in the vertical direction and that compresses the absorbent core in the thickness direction.

According to the underpants-shaped absorbent article, the stiffness increases locally in the portion of the absorbent core where the compressed portion is formed, making the absorbent core likely to deform integrally along the compressed portion. In other words, the left and right regions of the absorbent core (absorbent main body) separated by the basis-weight different portion becomes likely to be in conjunction with each other in the longitudinal direction (vertical direction). As a result, the regions of the absorbent main body on both lateral sides are likely to follow the movements of the wearer's body, and the fit is improved.

In such an underpants-shaped absorbent article, it is desirable that the belt portion has a planar elastic member that is capable of stretching and contracting along the leg opening.

According to such an underpants-shaped absorbent article, the stretchability which the planar elastic member exhibits makes the belt portion likely to closely fit the wearer's skin in surface-to-surface manner, along the leg opening. Therefore, even when the wearer walks or the like while wearing the underpants-shaped absorbent article, the underpants-shaped absorbent article is likely to maintain the fit in good condition.

In such an underpants-shaped absorbent article, it is desirable that the absorbent main body has a plurality of the basis-weight different portions that are arranged spacing in the lateral direction.

According to the underpants-shaped absorbent article, the gap between two laterally-adjacent basis-weight different portion makes a force less likely to be transmitted in the lateral direction. Furthermore, due to the stiffness difference that occurs in the gap between the basis-weight different portions, the gap serves as a bending point, and the absorbent main body becomes likely to bend and deform in the lateral direction. Through these things, the fit of the absorbent main body 10 can be improved.

In such an underpants-shaped absorbent article, it is desirable that, in the state where the absorbent main body is stretched, at least a part of the basis-weight different portion is located in a region above a vertical lower end of the joining portion.

According to the underpants-shaped absorbent article, when a force that pulls the belt portion outward in the lateral direction is applied, it is possible to efficiently suppress the lateral conjunctive motion of the absorbent main body, due to the presence of the basis-weight different portion in a region above the lower end of the joining portion, which is the region where the force is likely to act directly. Therefore, it is possible to suppress the deterioration of the fit of the absorbent main body.

In such an underpants-shaped absorbent article, it is desirable that at least a part of the basis-weight different portion is located in a region above the vertical lower end of the joining portion, on a back side in the front-back direction.

According to the underpants-shaped absorbent article, on the back side in the front-back direction, the absorbent main body, which has bent and deformed from the basis-weight different portion, fits into the intergluteal cleft, enabling to make it less likely to cause displacement or deformation in the lateral direction. This can make it more likely to suppress the deterioration of the fit of the absorbent main body.

In such an underpants-shaped absorbent article, it is desirable that the basis-weight different portion is located shifted downward beyond the vertical lower end of the joining portion, on a front side in the front-back direction.

According to the underpants-shaped absorbent article, there is less need to make the absorbent main body bend and deform because the wearer's body has smaller unevenness on the front side in the front-back direction than on the back side. Further, the wearer's excretion opening is located closer to the front (stomach) side. Therefore, by arranging the basis-weight different portion to be shifted downward beyond the lower end of the joining portion, it can make it likely to maintain the good fit and absorbency of the absorbent main body.

In such an underpants-shaped absorbent article, it is desirable that, in the state where the absorbent main body is stretched, at least a part of the basis-weight different portion is located in a region below a lower end of the belt portion in a lateral outer end portion.

According to the underpants-shaped absorbent article, if tacking occurs in the leg opening in a region that is located above the vertical lower end of the joining portion and below the lower end of the belt portion in the lateral outer end portion, the basis-weight different portion can suppress the effects of tacking from being transmitted in the lateral direction. This can make it more likely to suppress the deterioration of the fit of the absorbent main body.

In such an underpants-shaped absorbent article, it is desirable that the basis-weight different portion has a first inclined portion that is located on a back side and is inclined from below to above in the vertical direction and from inside to outside in the lateral direction, and that an absolute value of an angle formed by the first inclined portion and a horizontal direction is equal to or smaller than an absolute value of an angle formed by the joining portion and an upper end of the belt portion.

According to the underpants-shaped absorbent article, the inclination angle of the first inclined portion with respect to the horizontal direction is not excessively large, and this makes the absorbent main body likely to bend and deform in the vertical direction (longitudinal direction), making it possible to improve the fit of the absorbent main body along the gentle unevenness that continues from the upper buttocks to the back.

In such an underpants-shaped absorbent article, it is desirable that the basis-weight different portion has a second inclined portion that is located on a back side and is inclined from above to below in the vertical direction and from inside to outside in the lateral direction, and that an absolute value of an angle formed by the second inclined portion and a horizontal direction is equal to or smaller than an absolute value of an angle formed by the joining portion and an upper end of the belt portion.

According to the underpants-shaped absorbent article, the inclination angle of the second inclined portion with respect to the horizontal direction is not excessively large, and this makes the absorbent main body likely to bend and deform in the vertical direction (longitudinal direction), making it possible to improve the fit of the absorbent main body along the roundness of the lower buttocks.

### Embodiment

### Basic configuration of diaper 1

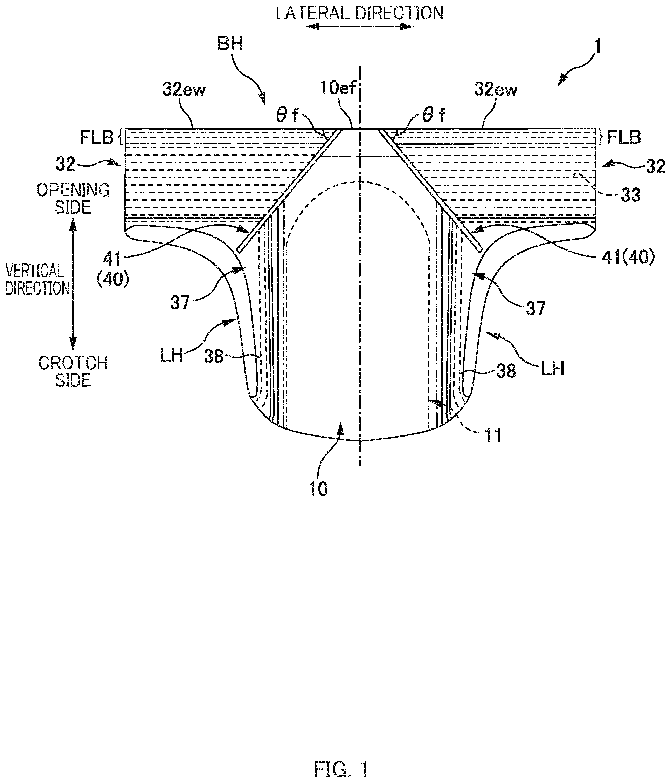

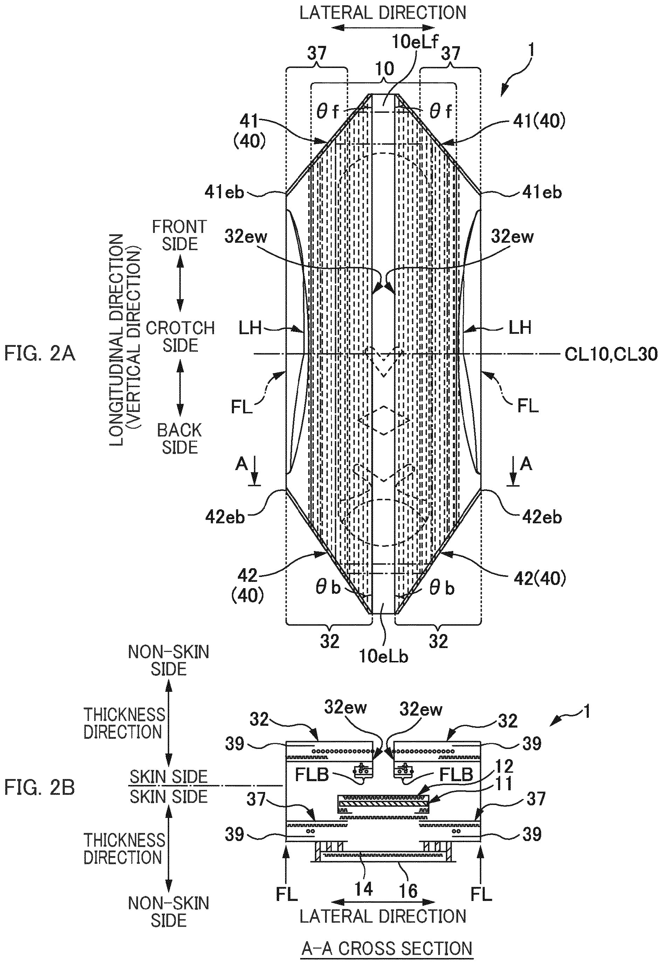

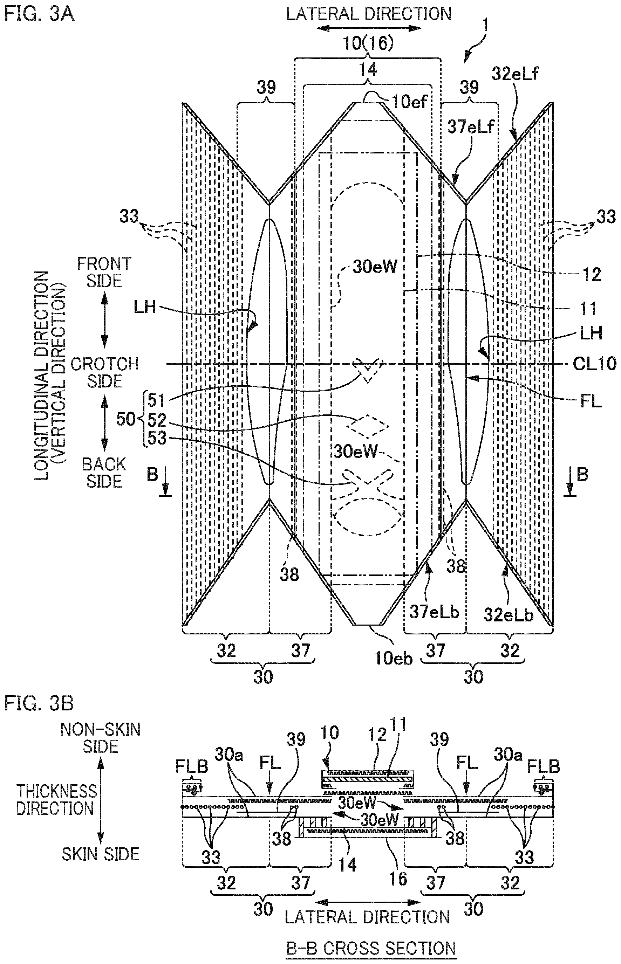

The following describes the basic configuration of an underpants-shaped disposable diaper 1 (hereinafter also referred to as "diaper 1") by way of example of an underpants-shaped absorbent article according to the present embodiment. FIG. 1 is a schematic front view of the diaper 1 as seen from the front side. FIG. 2A is a plan view of the diaper 1 in a state where an absorbent main body 10 and a belt portion 32 are stretched in the longitudinal direction. FIG. 2B is a cross-sectional view of the diaper 1 in the state shown in FIG. 2A, taken along a line A-A. FIG. 3A is a plan view of the diaper 1, which is unfolded by separating first joining portions 41 and second joining portions 42 in FIG. 2A. FIG. 3B is a cross-sectional view of the diaper 1 in the state shown in FIG. 3A, taken along a line B-B. Note that, in the cross-sectional views of FIGS. 2B and 3B, a later-described basis-weight different portion 50 is not shown.

The diaper 1 is in an underpants-shaped state as shown in FIG. 1 when being put on. The diaper 1 in an underpants-shaped state has a "vertical direction," a "lateral direction," and a "front-back direction," which are orthogonal to each other. Hereinafter, the upper side in the vertical direction is also referred to as the "waist opening side" or "opening side," and the lower side is also referred to as the "crotch side". Also, the front side in the front-back direction is referred to as a "front side" and the back side is referred to as a "back side."

The diaper 1 includes the waist opening BH, a pair of the leg openings LH and LH, the absorbent main body 10 extending along the vertical direction, a pair of the leg gather portions 37, and a pair of the belt portions 32 extending along the lateral direction.

The longitudinal (vertical) end portions 10eLf and10eLb of the absorbent main body 10 and the belt portions 32 arranged on two lateral sides are connected by corresponding joining portions 40 (the first joining portions 41 and the second joining portions 42). On the front side of the diaper 1, the end portion 10eLf of the absorbent main body 10 and the belt portions 32 are connected to a pair of the first joining portions 41 that incline from the waist opening BH toward the leg openings LH from above to below in the vertical direction and from inside to outside in the lateral direction. Similarly, on the back side, the end portion 10eLb of the absorbent main body 10 and the belt portions 32 are a pair of the second joining portions 42 incline from the waist opening BH toward the leg openings LH from above to below in the vertical direction and from inside to outside in the lateral direction. Further, the front end portions 37eLf of the leg gather portions 37 and the front portions 32eLf of the belt portions 32 are connected by the first joining portions 41, and the back end portions 37eLb of the leg gather portions 37 and the end portions 32eLb of the belt portions 32 are connected by the second joining portions 42. (See FIG. 3A). Examples of connecting methods using the first joining portion 41 and the second joining portions 42 include known connecting methods such as an adhesive, heat sealing, ultrasonic sealing, and combinations thereof.

Further, the first joining portions 41 of the diaper 1 are arranged so that the smaller one of angles with respect to the upper edge (inner end 32ew) of the belt portion 32 (inclination angle) is θf. Similarly, the second joining portions 42 are arranged so that the smaller one of angles with respect to the upper edge (inner end 32ew) of the belt portion 32 (inclination angle) is θb. In the present embodiment, the inclination angle θf of the first joining portion 41 and the inclination angle θb of the second joining portion 42 may be equal or different.

The end of the waist opening BH (opening-side end) of the diaper 1 is constituted by the front end 10ef and the back end 10eb of the absorbent main body 10 and the ends 32ew of the pair of belt portion. The end 10ef and end 10eb of the absorbent main body are provided in the central portion in the lateral direction.

In the belt portions 32 of the diaper 1, a plurality of waist elastic members 33 (made of elastic strings and the like) extending along the lateral direction are arranged side by side in the vertical direction (see FIG. 1), and give to the belt portions 32 a stretchability in the lateral direction, and give the fit to the waist opening BH. Further, the leg gather portions 37 and 37 are provided on both lateral sides of the absorbent main body 10. In each of the leg gather portions 37, leg elastic members 38 (made of elastic strings and the like) extending along the longitudinal direction of the absorbent main body 10 are arranged side by side in the lateral direction, and give to the leg gather portion 37 the stretchability in the longitudinal direction, and give the fit to the leg opening LH. Further, a stretchable sheet 39 is provided in a region spanning the belt portion 32 and the leg gather portion 37 (see FIG. 3A, and the like). The stretchable sheet 39 is a sheet member that can stretch and contract in the longitudinal direction of the absorbent main body 10, and is made of stretchable nonwoven fabric, stretchable film, or the like. As shown in FIG. 3A, the stretchable sheet 39 is arranged so as to surround the leg opening LH, and gives stretchability to the region. This makes the belt portions 32 and the leg gather portions 37 likely to fit to the wearer's skin in surface-to-surface manner, around the leg opening LH.

Furthermore, at the final stage of the manufacturing process, the diaper 1 is in a flat state, as shown in FIG.2. The diaper 1 in the flat state has three directions that are orthogonal to each other: a "longitudinal direction", a "lateral direction", and a "thickness direction". The longitudinal direction is a direction conforming to the longitudinal direction of the absorbent main body 10, and is a direction corresponding to the vertical direction in the underpants-shaped state (FIG. 1). In addition, in FIG. 2A, the state of "stretching in the longitudinal direction" (the stretched state) means a state where, when stretching the diaper 1 in the longitudinal direction in resistance to the contractive force by elastic members arranged extending along the longitudinal direction such as the waist elastic members 33 and the leg elastic members 38, the diaper is stretched to the extent that no wrinkles and gathers are seen in portions where the elastic members 33 or 38 are arranged. Therefore, the shape of the diaper 1 in the state of being stretched in the longitudinal direction is identical to the shape of the diaper 1 that is flatly extended in the state where the contractive force by the elastic members does not exhibit.

In the diaper 1 in the flat state shown in FIG. 2A, the longitudinal direction of the absorbent main body 10, the longitudinal direction of the pair of leg gather portions 37, and the longitudinal direction of the pair of belt portions 32 are aligned. In addition, the pair of belt portions 32 are overlaid on the skin side of the absorbent main body 10 and the pair of leg gather portions 37, and the pair of leg gather portions 37 and the pair of belt portions 32 extend in the longitudinal direction. The diaper 1 in the flat state shown in FIG. 2A is made into the underpants-shaped state (FIG. 1) as follows: the lateral inner ends 32ew of the belt portions 32 are opened outward in the lateral direction, and the absorbent main body 10 and the pair of leg gather portions 37 are folded one time at the center position CL10 of the diaper 1 in the longitudinal direction while the belt portions 32 being folded one time at their own center positions CL30 in the longitudinal direction.

As shown in FIG. 2A, the absorbent main body 10 is a sheet member whose end portions 10eLf and 10eLb in the longitudinal direction form a substantially V-like tapered shape in plan view, which is obtained by cutting a substantially rectangular member extending along the longitudinal direction along the first joining portions 41 and the second joining portions 42. As shown in FIG. 2B, in the absorbent main body 10, a liquid-permeable top sheet 12 made of nonwoven fabric, etc., an absorbent core 11, a liquid-impermeable back film 14 made of polyethylene film, polypropylene film, etc., and the back sheet 16 made of nonwoven fabric are overlaid in this order from the skin side in the thickness direction. Further, the absorbent main body 10 of the present embodiment is provided with the basis-weight different portion 50 (51 to 53), which will be described later (not shown in FIG. 2B).

The absorbent core 11 is a molded body obtained by molding a liquid absorbent material into a predetermined shape, and in the present embodiment, has an elliptical shape in which both longitudinal end portions are curved. Examples of the liquid absorbent material include the liquid-absorbent fiber (e.g., pulp fibers) and superabsorbent polymers, (so-called SAP). Further, the outer circumferential surface of the absorbent core 11 may be covered with the core-wrapping sheet (not shown). Examples of the core-wrapping sheet include a liquid permeable sheet such as tissue paper and nonwoven fabric.

The belt portion 32 and the leg gather portion 37 are formed by the same sheet member 30. As shown in FIGS. 3A and 3B, the sheet members 30 and 30 are respectively placed on both lateral sides of the absorbent main body 10. The sheet member 30 is one formed as follow: two flexible sheets 30a and 30a (e.g., nonwoven fabric sheets) are overlaid in the thickness direction, and between the sheets 30a, the waist elastic members 33, the leg elastic members 38, and the stretchable sheet 39 are fixed in a state of being stretched in the longitudinal direction. The lateral inner end portions 30eW and 30eW of the sheet members 30 and 30 are interposed between the top sheet 12 (absorbent core 11) and the back film 14 (back sheet 16), which constitute the absorbent main body 10, and are joined with the adhesive and the like.

Further, as shown in FIG. 3B, in the sheet member 30, a fold-back line FL extending along the longitudinal direction is set between a portion to be the belt portion 32 and a portion to be the leg gather portion 37. In each of the sheet members 30, the leg opening LH penetrates in the thickness direction so as to straddle the fold-back line FL in the lateral direction. Therefore, by folding the sheet member 30 inward in the lateral direction at the fold-back line FL, the belt portion 32 and the leg gather portion 37 are formed. Then, in the first joining portions 41 and 41 and the second joining portions 42 and 42, the end portions 10eLf and 10eLb of the absorbent main body 10 and the end portions 37eLf and 37eLb of the leg gather portions 37 are connected to the lateral end portions 32eLf and 32eLb of the belt portions 32, thus forming the diaper 1 in the flat state shown in FIG. 2A.

In addition, as shown in FIGS. 2B and 3B, the lateral outer end region of the belt portion 32 is provided with a folded-back portion FLB in which a part of the sheet member 30 is folded two times inward in the lateral direction and is fixed in a folded state with a hot-melt adhesive and the like. In the underpants-shaped diaper 1, the folded-back portion FLB is located at an end portion of the belt portion 32 on the opening side, and forms a part of the waist opening HB, and relieves stress caused by excessive tightening around the wearer's waist.

### Basis-weight different portion 50 of the absorbent main body 10

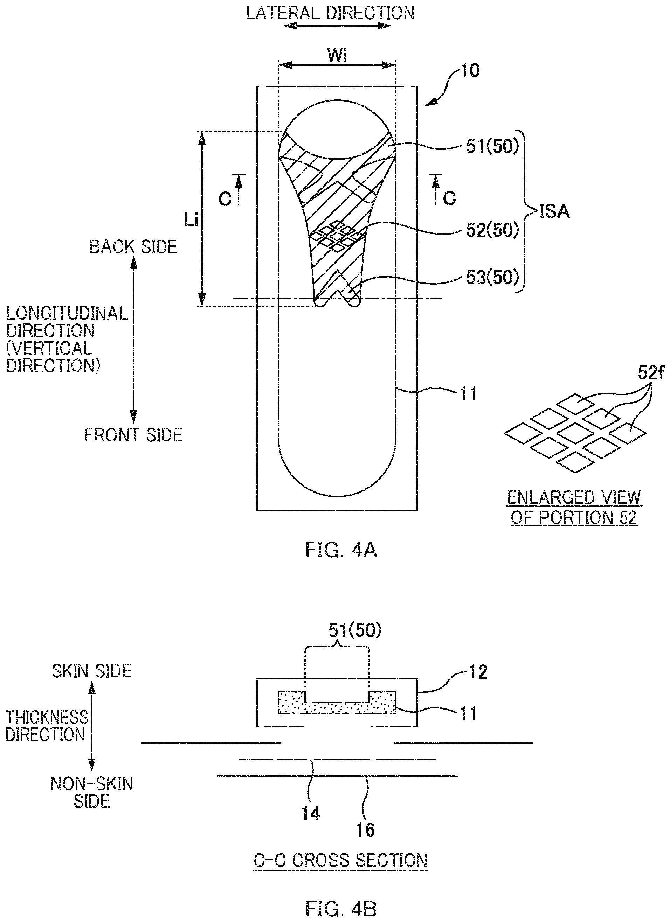

The following describes the basis-weight different portion 50 provided in the absorbent main body 10 of the diaper 1. FIG. 4A is a schematic plan view of the absorbent main body 10 in the stretched state as seen from the skin side. FIG. 4B is a cross-sectional view of the absorbent main body 10 taken along a line C-C in FIG. 4A.

The absorbent main body 10 of the present embodiment has the basis-weight different portion 50, which is a portion having a basis weight that is different when compared to those of portions adjacent in the lateral direction. In FIG. 4A, from the back side to the front side in the longitudinal direction, three basis-weight different portions 50 are arranged intermittently being spaced, namely a first basis-weight different portion 51, a second basis-weight different portion 52, and a third basis-weight different portion 53. The basis-weight different portion 50 is formed by increasing or decreasing the amount of the liquid absorbent material included per unit area in the absorbent main body 10, in a predetermined area of the absorbent main body 10. For example, in FIG. 4B, in the lateral central portion of the absorbent main body 10, by making the amount of the liquid absorbent material that constitutes the absorbent core 11 smaller than the portions adjacent thereto on both lateral sides, the basis-weight different portion 50 (the first basis-weight different portion 51) is formed. In other words, the basis-weight different portion 50 (the first basis-weight different portion 51) shown in FIG. 4B is a low-basis-weight portion, in which the total weight of pulp and the superabsorbent polymer (SAP) contained per the unit area of the absorbent main body 10 (absorbent core 11) is made smaller compared to the adjacent portions.

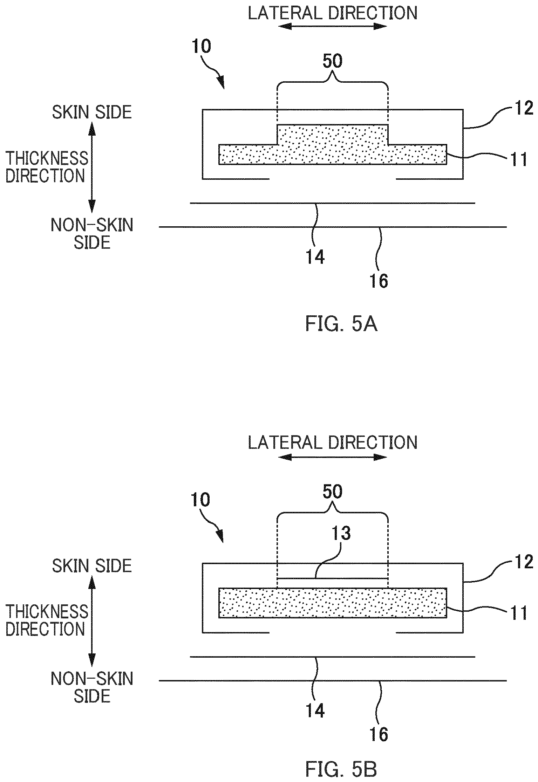

However, as shown in FIG. 4B, the basis-weight different portion 50 is not limited to the portion of the absorbent core 11 that has a smaller basis weight of the liquid absorbent material. FIGS. 5A and 5B are diagrams illustrating another example of the basis-weight different portion 50. In FIG. 5A, contrary to the case in FIG. 4B, in the lateral central portion of the absorbent main body 10, by making the total weight of the liquid absorbent material that constitutes the absorbent core 11 larger than the portions adjacent thereto both lateral sides, the basis-weight different portion 50 is formed. On the other hand, in FIG. 5B, the basis weight of the absorbent core 11 is uniform, but in the lateral central portion, between the top sheet 12 and the absorbent core 11 in the thickness direction, there is provided a sheet member 13 (second sheet 13) that includes absorbent resin (e.g., pulp or SAP), water-absorbent paper, water-absorbent nonwoven fabric, or the like. In this case as well, in the portion of the absorbent main body 10 where the second sheet 13 is provided, the basis-weight different portion 50 is formed in which the total weight of pulp, superabsorbent polymer (SAP) and the like that are contained in the unit area is large. In other words, the basis-weight different portion 50 shown in FIGS. 5A and 5B is a high-basis-weight portion, in which the total weight of pulp and the superabsorbent polymer (SAP) contained per the unit area of the absorbent main body 10 (absorbent core 11) is made larger compared to the adjacent portions.

Note that the basis-weight different portion 50 may be formed in a configuration other than that illustrated in FIGS. 4 and 5. In other words, it is sufficient that the basis weight in a predetermined region of the absorbent main body 10 is different from the basis weight of the regions adjacent thereto in the lateral direction. For example, in the absorbent main body 10 that includes a SAP sheet or so-called cellulose acetate tow in which SAP is hydrophilized, the basis-weight different portion 50 may be formed by increasing or decreasing their contents in a predetermined region.

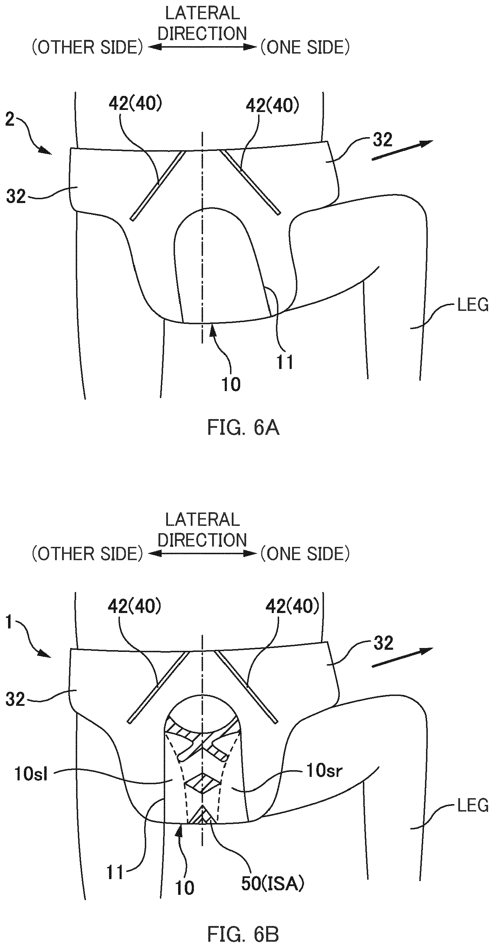

In the diaper 1 of the present embodiment, by providing the basis-weight different portion 50, it is possible to improve the fit of the absorbent main body 10 during usage. FIG. 6 is a diagram illustrating the difference in fit of the absorbent main body 10 during usage, between a conventional underpants-shaped diaper and the underpants-shaped diaper 1 of the present embodiment. FIG. 6A shows a conventional underpants-shaped diaper 2 of a comparative example when a wearer moves the wearer's body while putting on the diaper 2, as viewed from the wearer's back side (buttocks side). On the other hand, FIG. 6B shows the underpants-shaped diaper 1 of the present embodiment when a wearer moves the wearer's body while putting on the diaper 1, as viewed from the wearer's back side (buttock side).

In the diaper 2 of the comparative example shown in FIG. 6A, the basis-weight different portion 50 is not provided in the absorbent main body 10. The other configurations are substantially identical to the diaper 1 of the present embodiment. For example, while putting on such a diaper 2, when the wearer moves the lateral one-side leg (right leg) as shown in FIG. 6A, the belt portion 32 which is provided on the one side is pulled toward the one side according to the leg movement. Then, the absorbent main body 10, which is connected to the belt portion 32 located on the one side via the second joining portion 42, is pulled towards the one side in the lateral direction as a whole in conjunction with the belt portion 32. That is, if the absorbent main body 10 that fits to the wearer is displaced or deformed, there is a risk of impairing the fit of the absorbent main body 10.

In contrast, in the diaper 1 of the present embodiment, when the wearer moves lateral one-side leg (right leg) as shown in FIG. 6B, the belt portion 32 which is provided on the one side is pulled toward the one side according to the leg movement similarly to the case of FIG. 6A. Then, the absorbent main body 10, which is connected to the belt portion 32 located on the one side via the second joining portion 42, is pulled towards the one side in the lateral direction in conjunction with the belt portion 32. However, in the diaper 1, unlike the case in FIG. 6A, the lateral one-side portion 10sr of the absorbent main body 10 that is joined to the belt portion 32 located on the one side is pulled toward the one side, but the lateral other-side portion 10sl of the absorbent main body 10 is less likely to be pulled toward the one side. This is because the presence of the basis-weight different portion 50 in the absorbent main body 10 suppresses the conjunction of the lateral one-side portion (10sr) of the absorbent main body 10 and the lateral other-side portion (10sl) of the absorbent main body 10 across the basis-weight different portion 50.

The conventional diaper 2 has a configuration in which, when a force acts on the lateral one-side portion of the absorbent main body 10, the force is likely to be transmitted to the lateral other-side portion. In contrast, in the diaper 1 of the present embodiment, there is the basis-weight different portion 50 having a different basis weight on the midway of transmitting the force in the lateral direction, and it distributes or weakens the transmitted force. This makes the force less likely to be transmitted beyond the basis-weight different portion 50 to the opposite side in the lateral direction. Therefore, in FIG. 6B, even if the lateral one-side portion (10sr) of the absorbent main body 10 is pulled to the one side, the lateral other-side portion (10sl), which is located on the opposite side across the basis-weight different portion 50, is less likely to be pulled to the one side, and this can make it less likely to cause displacement or deformation. Thereby, when the diaper 1 is put on, it is possible to suppress the deterioration of the fit of the absorbent main body 10.

In particular, like the diaper 1 of the present embodiment, in the case of an underpants-shaped diaper that has a pair of belt portions 32 and 32 which are separated on the left and right sides, weakening the lateral conjunction of the absorbent main body 10 by the basis-weight different portion 50 makes it possible to independently deform the left and right portions of the absorbent main body 10 which are located across the basis-weight different portion 50. This makes the absorbent main body 10 likely to follow the movement of the wearer's body, which is effective for improving the fit.

Further, in the diaper 1, the conjunction-suppression region is formed by one or more basis-weight different portions 50, and this can make it likely to further suppress the lateral conjunction of the absorbent main body 10. In FIG. 4, the three basis-weight different portions 50, namely the first basis-weight different portion 51 to the third basis-weight different portion 53, are arranged in a distributed manner, forming a conjunction-suppression region ISA that covers a wide area of the absorbent core 11 (the region indicated by the hatched portion in FIG. 4A). It is desirable that the maximum value of the longitudinal length Li of the conjunction-suppression region ISA is larger than the maximum value of the lateral length (width) Wi thereof (Li > Wi). That is, it is desirable that the one or more basis-weight different portions 50 are provided in a wide area of the absorbent main body 10 that extends along the longitudinal direction (vertical direction).

Due to the formation of the conjunction-suppression region ISA in a wide area in the longitudinal direction (the vertical direction), the absorbent main body 10 becomes likely to be divided into lateral one-side portion (10sr in FIG. 6B) and the lateral other-side portion (10sl in FIG. 6B). That is, in the absorbent main body 10, the lateral one-side portion (10sr) and the lateral other-side portion (10sl) that are separated by the conjunction-suppression region ISA are likely to be widely formed in the longitudinal direction. As a result, the effects of deformation and displacement of the absorbent main body 10 are further less likely to be transmitted on the counter side in the lateral direction, and this can make it likely to suppress deterioration of the fit during usage.

Further, it is desirable that the basis-weight different portion 50 has a portion that is continuous in the longitudinal direction (vertical direction). In the case where the absorbent main body 10 has a thin, strip-shaped basis-weight different portion 50 extending along the lateral direction, the absorbent main body 10 is in a state where it is less likely to be divided in the lateral direction, so that the effect of suppressing the lateral conjunction of deformation or displacement significantly decreases. In contrast, in the present embodiment, each of the first to third basis-weight different portions 51 to 53 is configured to be continuous in the longitudinal direction (vertical direction) and have a predetermined length. As a result, at least in the portion where the first to third basis-weight different portions 51 to 53 are provided, it makes the lateral one-side portion (10sr) of the absorbent main body 10 and the lateral other-side portion (10sl) less likely to be in conjunction therewith. That is, since each of the basis-weight different portions 50 is continuous in the longitudinal direction (vertical direction), the left and right conjunction of the absorbent main body 10 is more likely to be suppressed compared to the case where the basis-weight different portions are not continuous. This suppresses the deterioration of the fit of the absorbent main body 10 when the diaper 1 is put on.

Further, in the case where the conjunction-suppression region ISA is composed of a plurality of the basis-weight different portions 50, it is preferable that the basis-weight different portions 50 are arranged spacing in the longitudinal direction (vertical direction). In the case of FIG. 4A, the first to third basis-weight different portions 51 to 53 are arranged intermittently at predetermined intervals in the longitudinal direction (vertical direction). As described above, it is desirable that the longitudinal (vertical) length Li of the conjunction-suppression region ISA, which is composed of the one or more basis-weight different portions 50, is long. However, in the case where the basis-weight different portions 50 constituting the conjunction-suppression region ISA are continuous within the length Li, this makes the absorbent main body 10 less likely to deform in the longitudinal direction. For example, the absorbent main body 10 is less likely to deform into a curved shape along the wearer's crotch, causing a risk of deterioration of the fit.

In contrast, in the absorbent main body 10 of the present embodiment, there are gaps between the first basis-weight different portion 51 to the third basis-weight different portion 53, which constitute the conjunction-suppression region ISA, and this makes the absorbent main body 10 likely to deform in the longitudinal direction (vertical direction) from the starting points of the bend, which are the stiffness differences that occur in the gaps. Therefore, in the diaper 1, the left and right conjunction of the absorbent main body 10 is suppressed in the region having the length Li in the longitudinal direction (vertical direction), and the absorbent main body 10 is likely to deform in the longitudinal direction (vertical direction). This makes the absorbent main body 10 likely to flexibly deform according to projections/recessions of the wearer's body, and the fit can be improved.

Further, in the case where the conjunction-suppression region ISA is composed of a plurality of the basis-weight different portions 50, the basis-weight different portions 50 may be arranged spacing in the lateral direction. For example, the second basis-weight different portion 52 provided in the absorbent main body 10 is formed by lining up a plurality of small the basis-weight different portions 52f at intervals with the vertical direction and the lateral direction, as shown in the enlarged view of the portion 52 in FIG. 4. With this configuration, in the second basis-weight different portion 52, a force is less likely to be transmitted in the lateral direction due to a gap between two laterally-adjacent basis-weight different portions 52f and 52f, and this makes the effects of deformation or displacement of the absorbent main body 10 less likely to be transmitted in the lateral direction. Further, due to the stiffness difference that occurs in the gap between the basis-weight different portions 52f and 52f, the gap serves as a bending point, and the absorbent main body 10 becomes likely to bend and deform in the lateral direction. Through these things, the fit of the absorbent main body 10 can be improved.

As mentioned above, in a predetermined region of the absorbent main body 10, the basis-weight different portion 50 is a region where the total basis weight (weight per the unit area) of pulp and superabsorbent polymer which constitute the absorbent main body 10 is smaller or larger than other portions which are laterally adjacent thereto. In the case where the basis-weight different portion 50 is provided in the central portion in the lateral direction, that is, in the case where the basis weight of the liquid absorbent material is higher or lower in the lateral central portion of the absorbent main body 10 than the basis weight on both sides thereof, it is possible to further improve the fit of the absorbent main body 10.

For example, the second basis-weight different portion 52 and the third basis-weight different portion 53 shown in FIG. 4 are located in the lateral central portion on the back side of the absorbent main body 10 in the front-back direction. The portions where the second basis-weight different portion 52 and the third basis-weight different portion 53 are arranged is portions that come into contact with the groove of the wearer's buttocks when the diaper 1 is put on. In this case, in the lateral central portion, the portion where the basis weight of the absorbent main body 10 has increased or decreased (the basis-weight different portion 50) serves as a bending point, making the absorbent main body 10 be folded one time in the lateral direction. This makes the absorbent main body 10 likely to fit into the groove of the wearer's buttocks and the excretion opening, and the like. This makes it possible to improve the fit of the absorbent main body 10 and simultaneously to makes excretion leakage less likely to occur.

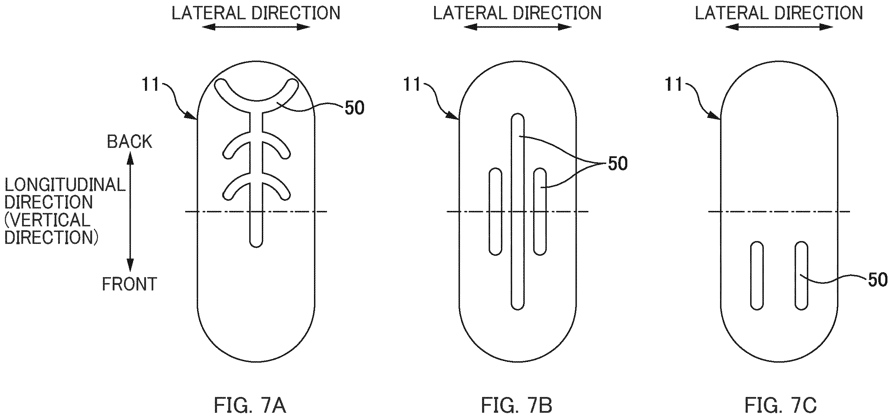

FIGS. 7Ato 7C are diagrams illustrating modified examples of the basis-weight different portion 50. The basis-weight different portion 50 may be modified as in FIGS. 7A to 7C, in addition to what is shown in FIG. 4. In FIG. 7A, the basis-weight different portion 50 is constituted by a straight-line-shaped portion extending along the longitudinal direction and curved portions extending from the straight-line-shaped portion to both lateral sides. The basis-weight different portion 50 has a shape like one in which the first to third basis-weight different portions 51 to 53 in FIG. 4 are integrally formed, and effects similar to those described above is obtained. Furthermore, if the basis-weight different portion 50 shown in FIG. 7A is divided at predetermined locations in the longitudinal direction, the absorbent main body 10 is likely to bend and deform in the longitudinal direction as described above, making it possible to improve the fit. In FIG. 7B, the basis-weight different portion 50 is constituted by a straight-line-shaped portion that is locate in the widthwise central portion and is elongated in the longitudinal direction and shorter straight-line-shaped portions that are placed on both sides of the straight-line-shaped portion. In this case, the effects of the deformation and displacement of the absorbent main body 10 is less likely to be transmitted in the lateral direction, and simultaneously it is more likely to form bending points where the absorbent main body 10 starts to bend in the lateral direction. This makes it possible to improve the fit when the absorbent main body 10 is sandwiched with the groin portion, in the crotch portion. In FIG. 7C, on the front side in the longitudinal direction, a pair of basis-weight different portions 50 each of which has a straight-line shape and extend briefly in the longitudinal direction are provided on the left and right sides. In this case, the effects of deformation and displacement of the absorbent main body 10 is less likely to be transmitted in the lateral direction, and simultaneously it is more likely to form bending points where the absorbent main body 10 starts to bend in the lateral direction, on both sides of the front portion. Therefore, when the wearer moves a leg forward at the time of walking and the like, deformation of the absorbent main body 10 to follow the movement of the legs makes it possible to improve the fit.

Note that the basis-weight different portion 50 can have a configuration other than the modified examples shown in FIGS. 7A to 7C as long as, as mentioned above, the effects of deformation and displacement of the absorbent main body 10 is less likely to be transmitted in the lateral direction because the basis weight of the liquid absorbent material that constitutes the absorbent main body 10 is different in the basis-weight different portion 50 compared to the portions adjacent thereto in the lateral direction. Alternatively, a configuration combining these may be used.

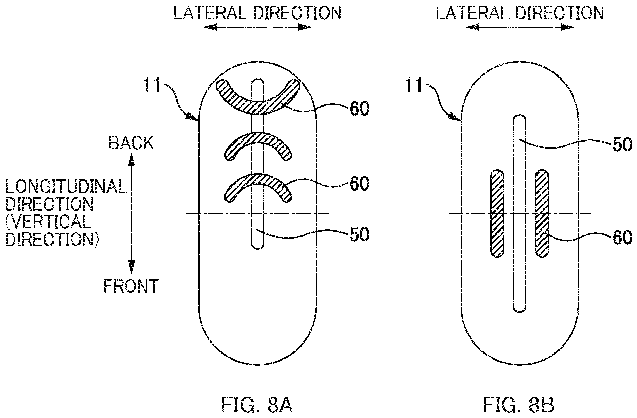

Further, in addition to the basis-weight different portion 50, the compressed portion 60 may be provided that compresses a predetermined region of the absorbent core 11 (the absorbent main body 10) in the thickness direction. FIGS. 8A and 8B are diagrams illustrating examples of a case in which the compressed portions 60 are provided in the absorbent main body 10. In FIG. 8A, a plurality of the compressed portions 60 are provided extending on both lateral sides from a straight-line-shaped basis-weight different portion 50 that extends in the longitudinal direction. Further, in FIG. 8B, a pair of straight-line-shaped compressed portions 60 and 60 are provided on both lateral sides of a straight-line-shaped basis-weight different portion 50 that extends in the longitudinal direction. In both cases, the compressed portion 60 having a predetermined length in the longitudinal direction (vertical direction) is provided on both lateral sides across the basis-weight different portion 50. The stiffness increases locally in portions of the absorbent core 11 where the compressed portion 60 is formed, and this makes the absorbent core 11 likely to deform integrally along the compressed portion 60. That is, although it suppresses the lateral conjunction of the absorbent core 11 (absorbent main body 10) across the basis-weight different portion 50, the left and right regions separated by the basis-weight different portions 50 are more likely to be in conjunction with each other in longitudinal direction (vertical direction). As a result, the regions of the absorbent main body 10 on both lateral sides are likely to follow the movements of the wearer's body, and the fit is improved.

Further, the belt portions 32 of the diaper 1 are each provided with the stretchable sheet 39, which is an elastic member having a planar shape that can stretch and contract along the leg opening LH. The stretchability exhibited by the stretchable sheet 39 makes the belt portion 32 (and the leg gather portion 37) likely to fit closely to the wearer's skin in surface-to-surface manner, around the leg opening LH. Therefore, even when the wearer walks or the like while wearing the diaper 1, the diaper 1 is likely to maintain the fit in good condition. In other words, even if depressions occur near the gluteus medius alternately on the left and right sides at the time of walking and the like, the belt portions 32 can be brought into surface-to-surface contact with the wearer's skin independently on the left and right sides near the leg opening LH. This makes the wearer less likely to feel discomfort such as rising in the depressions.

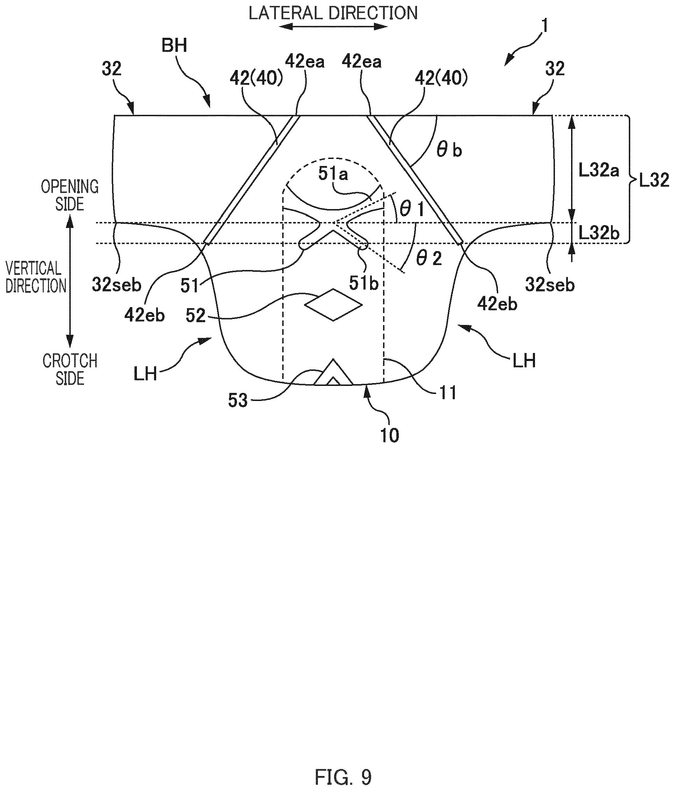

FIG. 9 is a schematic plan view illustrating the arrangement of the basis-weight different portion 50 on the back side of the diaper 1. FIG. 9 shows the underpants-shaped diaper 1 in the stretched state as viewed from the back side.

It is desirable that, when the diaper 1 is in the stretched state, at least a part of the basis-weight different portion 50 is provided above the vertical lower ends of the joining portions 40. In FIG. 9, a part of the first basis-weight different portion 51 is arranged in a region above the lower end 42eb of the second joining portion 42 on the back side of the diaper 1. When putting on the diaper 1, an operation is performed to fit the pair of belt portions 32 around the wearer's waist while spreading the waist opening BH by pulling the belt portions 32 toward both lateral sides. At this time, a force directed outward in the lateral direction acts on a region of the absorbent main body 10 that is joined to the belt portions 32 by the joining portions 40. That is, the region between the upper end 42ea and lower end 42eb of the second joining portion 42 (the joining portion 40) in the vertical direction (the region indicated by a symbol L32 in FIG. 9) is pulled in the lateral direction. Therefore, within the region L32 in FIG. 9, there is a risk that the absorbent main body 10 move or deform in the lateral direction.

Therefore, by providing the basis-weight different portion 50 in a region that overlaps the region L32 with respect to the vertical direction (a region above the lower end 42eb of the second joining portion 42), it makes the effects of deformation and displacement of the absorbent main body 10 less likely to be transmitted in the lateral direction. In other words, when a force that pulls the belt portions 32 outward in the lateral direction is applied, it is possible to efficiently suppress the lateral conjunctive motion of the absorbent main body 10 in the region where the force is likely to act directly (the region above the lower end of the joining portion 40). Therefore, it is possible to suppress the deterioration of the fit of the absorbent main body 10.

In the diaper 1, at least on the back side in the front-back direction, a part of the first basis-weight different portion 51 is arranged in a region above the lower end 42eb of the second joining portion 42. Since the intergluteal cleft exists on the back side of the wearer's body, when putting on the diaper 1, the absorbent main body 10 is likely to bend and deform along the intergluteal cleft in the lateral direction from the basis-weight different portion 50 (the first basis-weight different portion 51). Therefore, on the back side in the front-back direction, the absorbent main body 10, which has bent and deformed from the basis-weight different portion 50, fits into the intergluteal cleft, enabling to make it less likely to cause displacement or deformation in the lateral direction. This can make it more likely to suppress the deterioration of the fit of the absorbent main body 10.

Further, it is more desirable that, when diaper 1 is in the stretched state, at least a part of the basis-weight different portion 50 is provided above the vertical lower end of the joining portion 40 and below the lower ends of the belt portions 32 outside in the lateral direction. In FIG. 9, a part of the first basis-weight different portion 51 is arranged in a region indicated by a symbol L32b that is located above the lower end 42eb of the second joining portion 42 on the back side of the diaper 1 and below the lower ends 32seb of the belt portions 32 outside in the lateral direction. This region L32b is a region that is pulled outward in the lateral direction via the belt portions 32 when putting on the diaper 1, but the force acting on the region L32b is weaker than a force acting on the region adjacent to and located above the region L32b (the region indicated by a symbol L32a in FIG. 9). This is because, in the belt portions 32, the region L32a is pulled directly outward in the lateral direction with being held by the wearer's hands, whereas the region L32b is difficult to hold by the wearer's hands, and is pulled outward in the lateral direction by following the region L32a being pulled. Therefore, within the belt portions 32, the tension acting on the region L32b is smaller than the tension acting on the region L32a. This causes a risk that, when putting on the diaper 1, within the region L32b, the tension difference causes tacking in the leg openings LH and throughout the region L32b in the lateral direction, or rolling up toward the non-skin side.

In contrast, like the present embodiment, in the case where the basis-weight different portion 50 is provided in the region L32b, even if tacking and the like occur on the one side in the lateral direction, the effects of such tacking is less likely to be transmitted to the other side in the lateral direction. This can make it more likely to suppress the deterioration of the fit of the absorbent main body 10.

Note that, on the front side in the front-back direction, the basis-weight different portion 50 does not necessarily have to be provided in a region above the joining portion 40 (lower end of the first joining portion 41). On the front side of the diaper 1, the basis-weight different portion 50 is not provided in the region above the lower end 41eb of the first joining portion 41 in the vertical direction, and the basis-weight different portion 50 is arranged shifted downward beyond the lower end 41eb of the first joining portion 41 (see FIG. 2A). Since the intergluteal cleft and the like do not exist on the front side of the wearer's body and the unevenness is smaller compared to the back side, there is no need to make the absorbent main body 10 bend and deform to fit the uneven shape of the body. In addition, since the wearer's excretion opening is located closer to the front (stomach) side in the front-back direction, it is desirable that the area of the basis-weight different portion 50 is as small as possible on the front side of the absorbent main body 10 in order not to reduce the absorbing amount of urine and the like. Therefore, on the front side of the diaper 1, by arranging the basis-weight different portion 50 to be shifted downward beyond the lower end 41eb of the first joining portion 41, it can make it likely to maintain the good fit and absorbency of the absorbent main body 10.

In addition, as shown in FIG. 9 and the like, the first basis-weight different portion 51 is formed into an approximately X-shape, and has a first inclined portion 51a and a second inclined portion 51b, the first inclined portion 51a being inclined from below to above in the vertical direction and from inside to outside in the lateral direction, the second inclined portion 51b being inclined from above to below in the vertical direction and from inside to outside in the lateral direction. In such a configuration, it is desirable that the absolute value of an inclination angle θ1 of the first inclined portion 51a with respect to the horizontal direction is equal to or smaller than the absolute value of the angle θb, which is the smaller of the angles formed by the upper edge of the belt portion 32 and the second joining portion 42 (θb ≥ θ1).

Of the first basis-weight different portion 51, the first inclined portion 51a has a function of making it likely to make the absorbent main body 10 bend and deform in the vertical direction on the wearer's back side so that the absorbent main body 10 fits the wearer's body along the gentle unevenness that continues from the upper buttocks to the back. In order to make the absorbent main body 10 likely to bend in the vertical direction (longitudinal direction), it is necessary to prevent the inclination angle θ1 of the first inclined portion 51a with respect to the horizontal direction from becoming excessively large. Therefore, if the inclination angle θ1 of the first inclined portion 51a with respect to the horizontal direction is larger than the angle θb formed by the upper edge of the belt portion 32 and the second joining portion 42, the inclination is excessively large and this causes a risk that the absorbent main body 10 is less likely to bend and deform in the vertical direction (longitudinal direction). In contrast, in the diaper 1 of the present embodiment, the inclination angle θ1 with respect to the horizontal direction of the first inclined portion 51a is smaller than the angle θb formed by the upper edge of the belt portion 32 and the second joining portion 42. This makes the absorbent main body 10 likely to bent and deform in the vertical direction (longitudinal direction), making it possible to improve the fit in the upper region of the wearer's buttocks.

Similarly, it is desirable that the absolute value of an inclination angle θ2 of the second inclined portion 51b of the first basis-weight different portion 51 with respect to the horizontal direction is equal to or smaller than the absolute value of the angle θb, which is the smaller of the angles formed by the upper edge of the belt portion 32 and the second joining portion 42 (θb ≥ θ2). Of the first basis-weight different portion 51, the second inclined portion 51b is arranged so as to be inclined on the opposite side to the first inclined portion 51a on the wearer's back side in order to fit the absorbent main body 10 along the roundness of the lower buttocks. At this time, in order to make the absorbent main body 10 likely to bend in the vertical direction (longitudinal direction), it is necessary to prevent the inclination angle θ2 of the second inclined portion 51b with respect to the horizontal direction from becoming excessively large. In the diaper 1 of the present embodiment, the inclination angle θ2 of the second inclined portion 51b with respect to the horizontal direction F51b is smaller than the angle θb formed by the upper edge of the belt portion 32 and the second joining portion 42. This makes the absorbent main body 10 likely to bent and deform in the vertical direction (longitudinal direction), making it possible to improve the fit along the roundness of the lower buttocks of the wearer.

### Other embodiments

Although the embodiments of the present disclosure have been described hereinabove, the above embodiments of the present disclosure are simply to facilitate understanding of the present disclosure and are not in any way to be construed as limiting the present disclosure. The present disclosure may variously be changed or altered without departing from its gist and encompass equivalents thereof. For example, the following modifications are possible.

In the above-mentioned the embodiment, the underpants-shaped diaper 1 is described as an example of the underpants-shaped absorbent article, but the present invention is also applicable to other underpants-shaped absorbent article except for underpants-shaped diapers. For example, it may be an underpants-shaped sanitary napkin (shorts-type sanitary napkin) for children or adults, an underpants-shaped (shorts-type) absorbent pad, and the like.

In the above-mentioned the embodiment, the elastic members that stretch and contract in the longitudinal direction may be provided in the central portion of the absorbent main body 10 in the lateral direction. For example, a plurality of the crotch elastic members (not shown), which are composed of elastic strings and the like, are arranged extending along the longitudinal direction on the skin side or the non-skin side with respect to the absorbent core 11. If such crotch elastic members are arranged, a high-stiffness portion is formed in portions where the absorbent main body 10 has contracted due to the contractive force of the crotch elastic members, and this realizes the same effect as the above-mentioned the basis-weight different portion 50. That is, the force acting on the lateral one side of the absorbent main body 10 is less likely to be transmitted to the opposite side in the lateral direction across the portion where the crotch elastic members are arranged (high-stiffness portion). This can makes the absorbent main body 10 less likely to displace or deform. This makes it possible to improve the fit of the absorbent main body 10 during usage.

### REFERENCE SIGNS LIST

1. diapers (underpants-shaped absorbent article),
10. the absorbent main body,
10ef, 10eb end, 10eLf, 10eLb end portion,
10sr part (one side), 10sl part (other side),
11. absorbent core,
12. top sheet, 13 second sheet,
14. back film, 16 back sheet,
30. sheet member, 30a sheet, 30eW end,
32. belt portion,
32ew end portion, 32eLf, 32eLb end portion, 32seb lower end,
33. waist elastic member (elastic string),
37. leg gather portion,
37eLf, 37eLb end portion,
38. leg elastic member (elastic string),
39. stretchable sheet,
40. joining portion,
41. first joining portion,
42. second joining portion, 42ea upper end, 42eb lower end,
50. basis-weight different portion,
51. first basis-weight different portion, 51a first inclined portion, 51b second inclined portion,
52. second basis-weight different portion, 52f basis-weight different portion,
53. third basis-weight different portion,
60. compressed portion,
ISA conjunction-suppression region,
θf angle, θ1 angle, θ2 angle,
BH waist opening, LH leg opening,
CL10 center position (absorbent main body)

## Claims

1. An underpants-shaped absorbent article having a vertical direction, a lateral direction, and a front-back direction that intersect with each other,
the underpants-shaped absorbent article comprising:
an absorbent main body that includes at least one of pulp and superabsorbent polymer; and
a belt portion that connects a front upper end portion and a back upper end portion of the absorbent main body,
the underpants-shaped absorbent article having a waist opening and a pair of leg openings,
the belt portion being joined to the absorbent main body by a joining portion that inclines from the waist opening towards the leg opening from above to below in the vertical direction and from inside to outside in the lateral direction,
in a state where the absorbent main body is stretched, the absorbent main body having a basis-weight different portion,
the basis-weight different portion being a portion in which a total basis weight of pulp and superabsorbent polymer that are contained per the unit area is different compared to a portion adjacent in the lateral direction.

2. The underpants-shaped absorbent article according to claim 1, wherein
the underpants-shaped absorbent article has a conjunction-suppression region that is composed of the one or more basis-weight different portion, and
a length of the conjunction-suppression region in the vertical direction is longer than a length of the conjunction-suppression region in the lateral direction.

3. The underpants-shaped absorbent article according to claim 1 or 2, wherein
the basis-weight different portion has a portion that is continuous in the vertical direction.

4. The underpants-shaped absorbent article according to any one of claims 1 to 3, wherein
the absorbent main body has a plurality of the basis-weight different portions that are arranged spacing in the vertical direction.

5. The underpants-shaped absorbent article according to any one of claims 1 to 4, wherein
the basis-weight different portion is provided in a central portion in the lateral direction, and
in the basis-weight different portion,
a total basis weight of pulp and superabsorbent polymer that are contained per unit area is higher than a portion that is adjacent to the central portion on both sides in the lateral direction.

6. The underpants-shaped absorbent article according to any one of claims 1 to 4, wherein
the basis-weight different portion is provided in a central portion in the lateral direction, and
in the basis-weight different portion,
a total basis weight of pulp and superabsorbent polymer that are contained per unit area is lower than a portion that is adjacent to the central portion on both sides in the lateral direction.

7. The underpants-shaped absorbent article according to any one of claims 1 to 6, wherein
the absorbent main body includes a liquid-absorbent absorbent core, and
a compressed portion is provided on each of two lateral sides of the basis-weight different portion,
the compressed portion being a portion that has a predetermined length in the vertical direction and that compresses the absorbent core in the thickness direction.

8. The underpants-shaped absorbent article according to any one of claims 1 to 6, wherein
the belt portion has a planar elastic member that is capable of stretching and contracting along the leg opening.

9. The underpants-shaped absorbent article according to any one of claims 1 to 8, wherein
the absorbent main body has a plurality of the basis-weight different portions that are arranged spacing in the lateral direction.

10. The underpants-shaped absorbent article according to any one of claims 1 to 9, wherein
in the state where the absorbent main body is stretched,
at least a part of the basis-weight different portion is located in a region above a vertical lower end of the joining portion.

11. The underpants-shaped absorbent article according to claim 10, wherein
at least a part of the basis-weight different portion is located in a region above the vertical lower end of the joining portion, on a back side in the front-back direction.

12. The underpants-shaped absorbent article according to claim 10 or 11, wherein
the basis-weight different portion is located shifted downward beyond the vertical lower end of the joining portion, on a front side in the front-back direction.

13. The underpants-shaped absorbent article according to any one of claims 10 to 12, wherein
in the state where the absorbent main body is stretched,
at least a part of the basis-weight different portion is located in a region below a lower end of the belt portion in a lateral outer end portion.

14. The underpants-shaped absorbent article according to any one of claims 1 to 13, wherein
the basis-weight different portion has a first inclined portion that is located on a back side and is inclined from below to above in the vertical direction and from inside to outside in the lateral direction, and
an absolute value of an angle formed by the first inclined portion and a horizontal direction is equal to or smaller than an absolute value of an angle formed by the joining portion and an upper end of the belt portion.

15. The underpants-shaped absorbent article according to any one of claims 1 to 14, wherein
the basis-weight different portion has a second inclined portion that is located on a back side and is inclined from above to below in the vertical direction and from inside to outside in the lateral direction, and
an absolute value of an angle formed by the second inclined portion and a horizontal direction is equal to or smaller than an absolute value of an angle formed by the joining portion and an upper end of the belt portion.
